# EUROPEAN PATENT APPLICATION

(11) **EP 3 895 733 A1**
(43) Date of publication of application: **20.10.2021**
(21) Application number: 19895550.2
(22) Date of filing: 11.12.2019
(51) Int. Cl.: A61K 39/395, A61K 45/00, A61P 35/00, C12Q 1/6886, G01N 33/15, G01N 33/50, G01N 33/68

(54) **METHOD FOR FORECASTING ARRIVAL OF DRUG INSIDE DISEASED TISSUE**

(30) Priority: 14.12.2018 JP 2018234097
(71) Applicant: KONICA MINOLTA, INC., Tokyo 100-7015 (JP); National Cancer Center, Tokyo 104-0045 (JP)
(72) Inventor: HAMADA, Akinobu, Tokyo 104-0045 (JP); HAYASHI, Mitsuhiro, Tokyo 104-0045 (JP)
(74) Representative: Henkel & Partner mbB
(86) International application number: PCT/JP2019/048415
(87) International publication number: WO 2020/122102

(57) **Abstract**

Provided is a method for forecasting arrival of a drug inside a diseased tissue before administration of the drug to a patient. The present invention includes a method for forecasting, in a diseased tissue in which a biomarker A derived from a diseased cell is expressed, arrival of a drug targeting the biomarker A inside the diseased tissue, the method including a step B for acquiring information on expression of a biomarker B derived from a non-diseased cell adjacent to or close to the diseased cell.

## Description

### Technical Field

The present invention relates to a method for forecasting arrival of a drug inside a diseased tissue.

### Background Art

In conventional medical treatment, when a disease name is determined based on medical information such as general interviews, physical findings, or biochemical tests, a therapeutic drug corresponding to the disease name is provided. However, at this time, the drug may be effective or ineffective for a patient depending on an individual constitution and a genetic difference between patients, and side effects may appear. In addition, even if the same disease name is determined, the state of a disease varies depending on an individual, and therefore a therapeutic drug suitable for the constitution of a patient is preferably applied.

Under such circumstances, personalized medical treatment that provides optimal medical treatment in consideration of a difference between individuals in effects of a drug has become widespread. By grasping a genetic background, a physiological condition, a disease condition, and the like of a patient with a biomarker in addition to general diagnostic information, an appropriate treatment method for each patient is being provided.

Examples of the biomarker include a diagnostic marker used for diagnosing a disease, a prognostic marker that forecasts a course of a disease that does not depend on a specific treatment, a pharmacodynamic marker that indicates a mechanism of an action of a drug, a forecast marker that forecasts an effect of a specific treatment, a surrogate marker that substitutes for a true endpoint of a clinical trial, a safety/toxicity marker that evaluates safety and toxicity of a drug, and a stratification marker that selects a patient expressing a specific molecule related to a drug.

If a target molecule is clear, patients can be stratified using a stratification marker for the target molecule. For example, Patent Literature 1 discloses that it is determined whether a subject is affected by depression based on an expression profile of a depression marker gene, and then the type of depression is identified based on the expression profile of a stratification marker gene. Patent Literature 2 discloses that a potential candidate protein/peptide biomarker is selected qualitatively and quantitatively from a proteome in a sample of healthy or cancerous non-human mammal-derived tissues, serum, plasma, or the like by predetermined analysis, a protein/peptide biomarker is selected, for example, by affinity analysis between the selected candidate protein/peptide biomarker and a proteome in a sample of healthy or cancerous human-derived tissues, serum, plasma, or the like, and a diagnosis of prostate cancer, patient stratification, and the like can be performed using the protein/peptide biomarker.

Meanwhile, in chemotherapy for a diseased tissue, it is necessary to select a highly sensitive drug and sufficiently cause the drug to reach the tissue as a condition of administration in which an effect of the chemotherapy is exhibited. However, depending on a component of the drug, the effect may be weakened by an action of gastric acid or the like, or the required amount of the drug does not necessarily reach the diseased tissue due to metabolism in the liver or the like. In addition, even if the drug is absorbed and reaches a target tissue after systemic circulation, a barrier of affinity with a target cell affects expression of the effect. In addition, the drug that has reached a normal tissue is one of causes of expressing side effects as an off-target.

Drug distribution in the heart, brain, lungs, and the like, which are normal tissues called off-target (non-target), is a cause of a serious adverse effect. Therefore, a ratio between efficient drug delivery in a target and drug delivery in a non-target is considered to indicate an important meaning in selecting an optimal drug.

In order to solve this problem, a technique for efficiently delivering a drug to a diseased tissue has been studied, and various drug delivery system (DDS) preparations have been developed. However, for example, for an intractable cancer such as pancreatic cancer or scirrhous gastric cancer, many DDS preparations do not have ideal therapeutic effects. One of reasons therefor is that cells derived from a normal tissue act as a barrier around a cancer tissue, and a characteristically thick stromal tissue/fibroblast and the like are formed. It has been clarified that many DDSs cannot break through this stroma, remain in the vicinity of blood vessels around a cancer tissue, and cannot be delivered into the cancer tissue (Non Patent Literature 1).

### Citation List

### Patent Literature

Patent Literature 1: JP 2005-312435 A
Patent Literature 2: JP 2011-521215 A

### Non Patent Literature

Non Patent Literature 1: Nature Nanotechnology. 2011 (12): pp 815-823.

### Summary of Invention

### Technical Problem

As described above, even if a drug or a preparation effective for treating a disease is administered to a patient, the drug or the preparation does not arrive inside a target diseased tissue, and therefore an appropriate effect cannot be necessarily obtained. Therefore, in order to obtain an appropriate therapeutic effect, it is necessary to repeat trial and error, such as increasing a dose of the drug, changing the drug to another drug, or using the drug in combination with another drug. This imposes a physical burden due to side effects of the drug, continuation of long-term treatment, and the like on the patient. Therefore, use of a biomarker is required as a method for evaluating and forecasting arrival of a drug inside a diseased tissue in advance.

The present invention has been achieved in view of this situation, and an object of the present invention is to provide a method for forecasting arrival of a drug at a target inside a diseased tissue from information on a predetermined biomarker before administering the drug to a patient. Another object of the present invention is to provide a method for screening a new drug that specifically acts on a disease from the forecasted information.

### Solution to Problem

The present invention includes the following inventions [1] to [8] in order to solve the above problems.
[1] A method for forecasting, in a diseased tissue in which a biomarker A derived from a diseased cell is expressed, arrival of a drug targeting the biomarker A inside the diseased tissue, the method including a step B for acquiring information on expression of a biomarker B derived from a non-diseased cell adjacent to or close to the diseased cell.
[2] A method for forecasting arrival of a drug targeting a biomarker A derived from a diseased cell in a diseased tissue inside the diseased tissue from both of information on expression of the biomarker A and information on expression of a biomarker B derived from a non-diseased cell adjacent to or close to the diseased cell, the method including: a step A for acquiring the information on expression of the biomarker A; and a step B for acquiring the information on expression of the biomarker B.
[3] The method according to [1] or [2], in which the diseased tissue is a cancer tissue, and the diseased cell is a cancer cell.
[4] The method according to any one of [1] to [3], in which the biomarker A is at least one selected from the group consisting of an immune protein expressed in a cancer cell, a pathway protein expressed in a cancer cell, and a progression protein expressed in a cancer cell.
[5] The method according to any one of [1] to [4], in which the biomarker B is a protein involved in an interferon signaling pathway and is at least one selected from the group consisting of BST2, OAS1, OAS2, OAS3, IFIT1, IFIT2, XAF1, clusterin, DCLK1, and MX1.
[6] The method according to any one of [1] to [5], in which the drug is a molecular-targeted drug.
[7] A method for screening a drug targeting a biomarker A derived from a diseased cell in a diseased tissue based on the method for forecasting arrival of the drug targeting the biomarker A inside the diseased tissue according to any one of [1] to [6].
[8] An activity regulator of a biomarker B derived from a non-diseased cell adjacent to or close to a diseased cell.
[9] A pharmaceutical composition containing a drug targeting a biomarker A derived from a diseased cell in a diseased tissue and the activity regulator according to [8].

### Advantageous Effects of Invention

According to the present invention, by acquiring and analyzing information on expression of a biomarker of a patient's diseased tissue before administering a drug, arrival of the drug at a target in the diseased tissue can be forecasted, and a therapeutic effect of the drug can be forecasted. In addition, the drug can be screened from information forecasting arrival of the drug. In addition, by regulating activity of the biomarker B, a therapeutic effect of the drug can be enhanced.

### Brief Description of Drawings

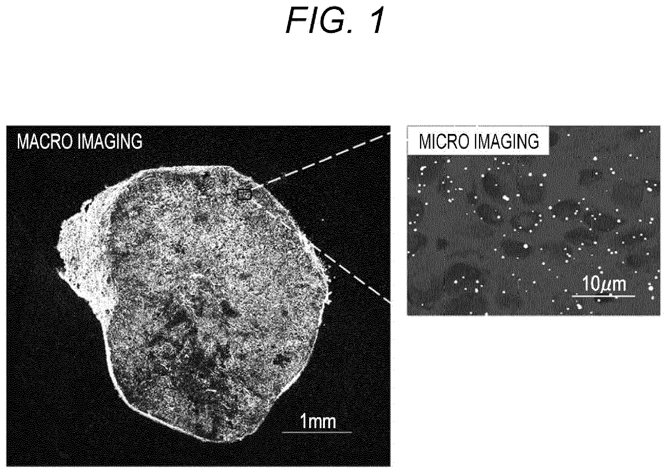

Fig. 1 is an image photograph of a fluorescence image when pharmacokinetics of a living tissue in a microenvironment is analyzed by micro-pharmacokinetics (PK).

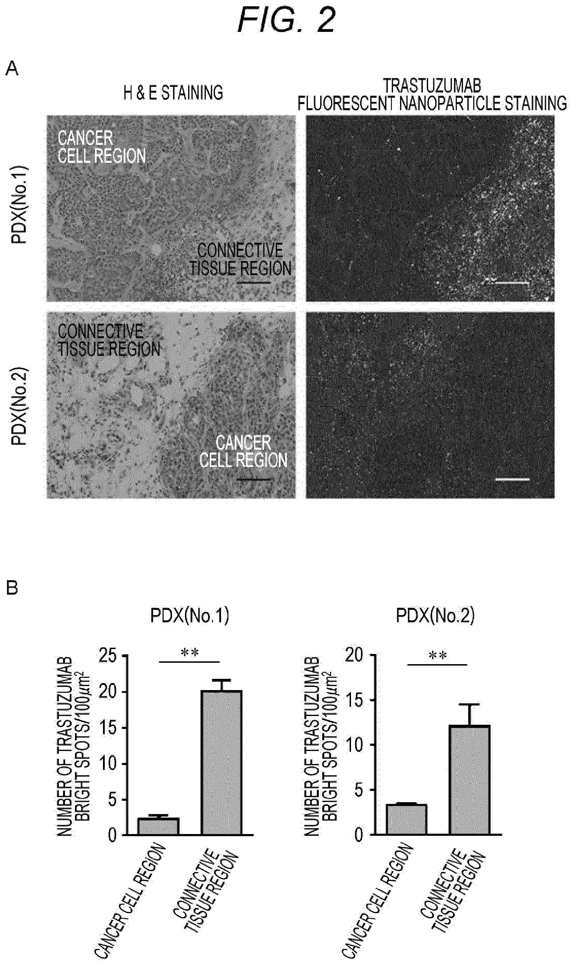

Fig. 2A illustrates typical micrographs of patient derived xenograft (PDX) specimen samples (No. 1 and No. 2) stained with hematoxylin and eosin (H & E staining) and stained with an anti-trastuzumab antibody labeled with high-brightness fluorescent nanoparticles (trastuzumab fluorescent nanoparticle staining). Fig. 2B is a graph illustrating the number of bright spots of high-brightness fluorescent nanoparticles per 100 µm² of a specimen labeled with the anti-trastuzumab antibody in a cancer cell region and a connective tissue region of each of the two specimen samples.

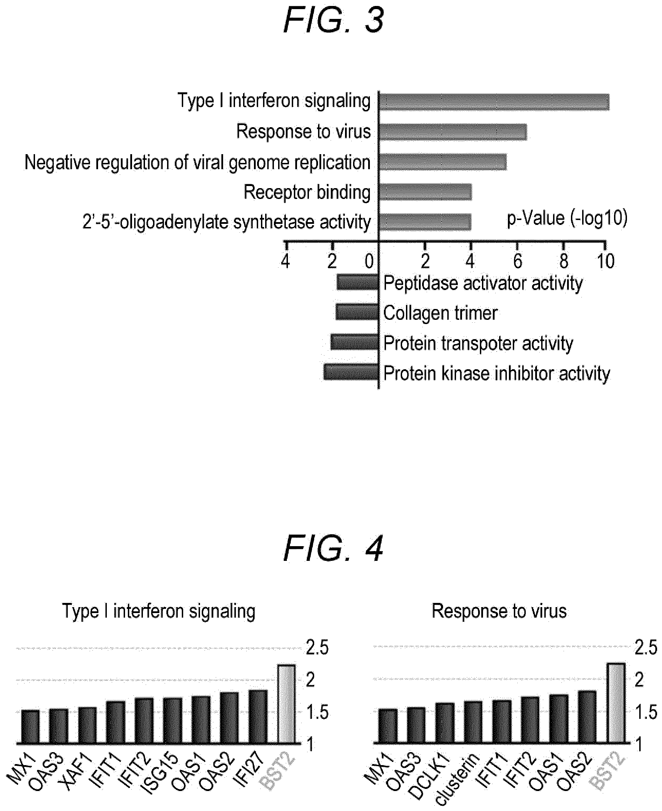

Fig. 3 is a graph illustrating results of GO Terms after GO analysis of a gene expressed in a non-diseased tissue adjacent to a region (trastuzumab low arrival region) with a small number of bright spots of particles that have arrived inside a diseased tissue in trastuzumab fluorescent nanoparticle staining for the trastuzumab-administered PDX specimen sample (No. 2).
Fig. 4 is a graph illustrating expression levels of major genes for the two GO Terms in Fig. 3.

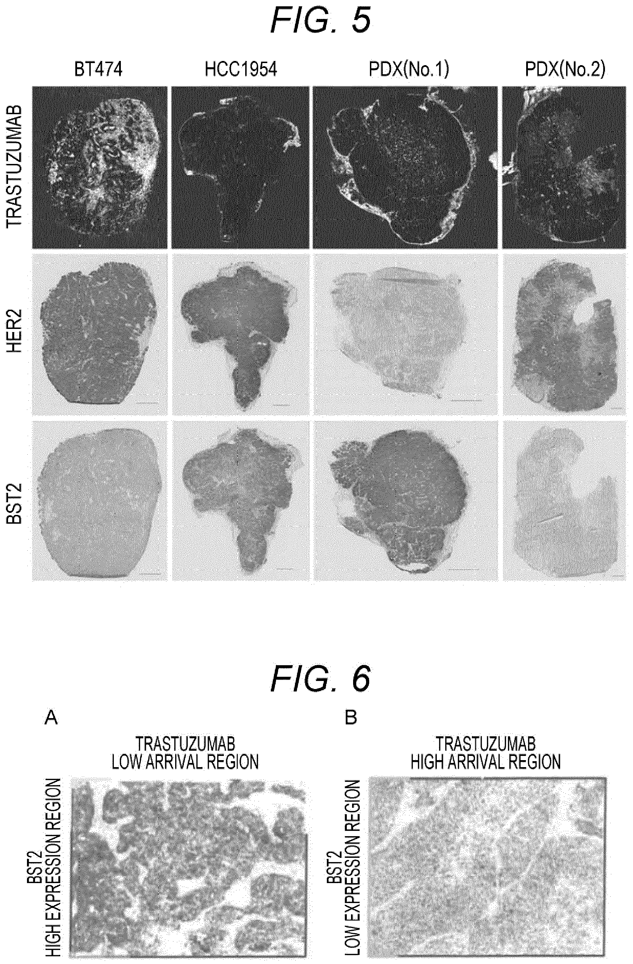

Fig. 5 illustrates typical micrographs of two types of cell line-derived xenograft (CDX) specimen samples (BT474 and HCC1954) and two types of PDX specimen samples (NO. 1 and NO. 2) treated with an anti-trastuzumab antibody labeled with high-brightness fluorescent nanoparticles (trastuzumab), an anti-HER2 antibody (HER2), and an anti-BST2 antibody (BST2), in immunohistochemical observation.
Figs. 6A and 6B are typical micrographs illustrating a relationship between a difference in trastuzumab arrival and a difference in BST2 expression.

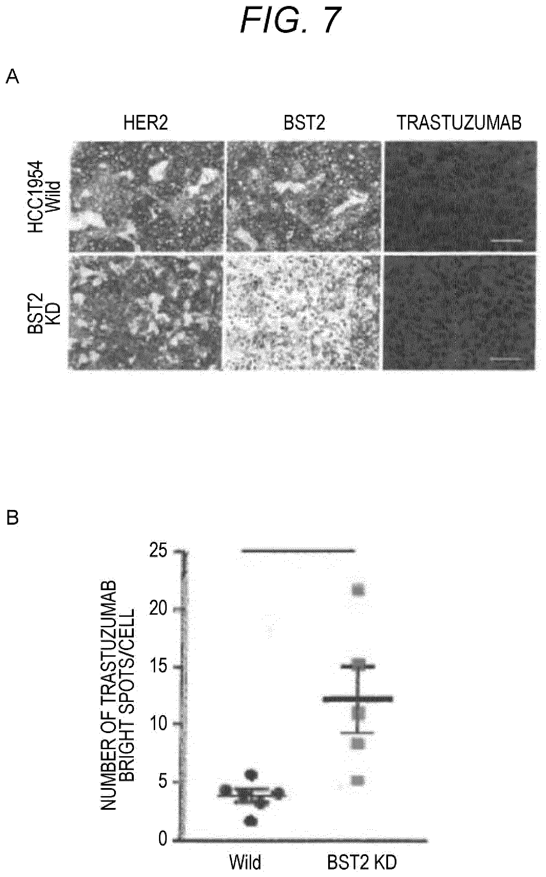

Fig. 7A illustrates typical micrographs of tumor tissues prepared from a BST2 knockout cell and HCC1954 treated with an anti-HER2 antibody, an anti-BST2 antibody, and an anti-trastuzumab antibody labeled with high-brightness fluorescent nanoparticles in immunohistochemical observation. Fig. 7B is a graph illustrating a quantitative value of trastuzumab arrival inside the cell.

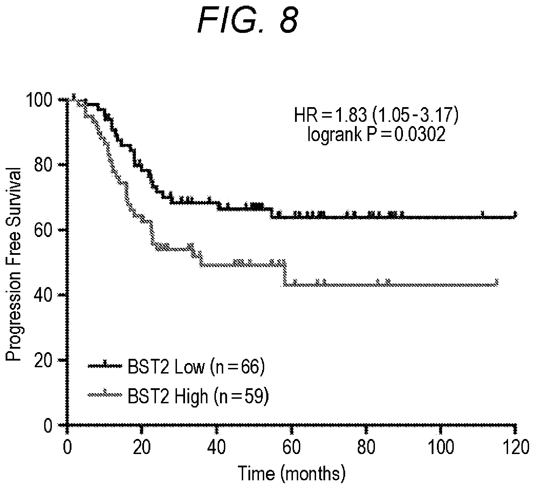

Fig. 8 is a graph illustrating progression-free survival ratios (%) of breast cancer patients due to a difference in BST2 expression. The upper line illustrates a progression-free survival ratio of a patient with a low BST2 expression level, and the lower line illustrates a progression-free survival ratio of a patient with a high BST2 expression level.

### Description of Embodiments

The present invention will be described in detail below.

As described above, the present invention relates to a method for forecasting, in a diseased tissue in which a biomarker A derived from a diseased cell is expressed, arrival of a drug targeting the biomarker A at a target inside the diseased tissue, the method including a step B for acquiring information on expression of a biomarker B derived from a non-diseased cell adjacent to or close to the diseased cell.

### <Diseased tissue>

In the present invention, the "diseased tissue" generally means a tissue that changes with onset or progression of a disease, and may include not only a diseased cell but also a normal cell such as stroma around the diseased cell. The diseased tissue includes, for example, a tumor tissue. The tumor usually refers to a malignant tumor, and includes: cancer or carcinoma which is a malignant tumor generated from an epithelial cell such as the skin, stomach, or intestinal mucosa; "sarcoma" which is a malignant tumor generated from a non-epithelial cell such as the muscle, fiber, bone, fat, blood vessel, or nerve; and leukemia and malignant lymphoma generated from a hematopoietic organ.

Examples of the tumor include a solid cancer such as a cell tumor, melanoma, sarcoma, a brain tumor, a head and neck cancer, a stomach cancer, a lung cancer, a breast cancer, a liver cancer, a colon cancer, a cervical cancer, a prostate cancer, or a bladder cancer, leukemia, lymphoma, and multiple myeloma.

### <Biomarker>

### (Biomarker A)

The biomarker A derived from a diseased cell in a diseased tissue is a biological substance such as a protein or a nucleic acid specifically expressed in a diseased cell in a diseased tissue collected from a patient. By performing gene analysis of the diseased cell, the biomarker A can be identified based on mutation information in the gene analysis. The biomarker A is not particularly limited as long as being expressed in a diseased cell, and one type of biological substance may be selected to be used as the biomarker A, or two or more types of biological substances may be selected to be used as the biomarker A.

When the biomarker A is a nucleic acid, the biomarker A is preferably any one of various RNAs such as mRNA, tRNA, siRNA, and non-cording-RNA derived from a genome in a diseased cell or stroma, and is preferably a miRNA such as miR21, miR34a, miR197, miR200, miR513., miR-133a, miR-143, exosomal micro-RNA (miR-181c, miR-27b), let-7a, miR-122, or iR4717.

When the diseased tissue is cancer tissue, the biomarker A is preferably a cancer-related protein. Examples of the cancer-related protein include an immune protein expressed in a cancer cell, a pathway protein expressed in a cancer cell, and a progression protein expressed in a cancer cell. As these cancer-related proteins, various proteins are known. An appropriate protein can be selected according to a purpose of diagnosis or treatment, a mechanism of action of a drug to be used, and the like without being particularly limited. For example, proteins encoded by genes of an immune gene panel, a pathway gene panel, and a progression gene panel included in a cancer-related gene expression panel provided by nCounter correspond to an immune protein, a pathway protein, and a progression protein expressed in a cancer cell, respectively. Mutant proteins corresponding to mutant genes of these genes can also be included in the immune protein, the pathway protein, and the progression protein, respectively.

Examples of the immune protein expressed in a cancer cell include CD40, TL1A, GITR-L, 4-188-L, CX4D-L, CD70, HHLA2, ICOS-L, CD85, CD86, etc. CD80, MHC-II, PDL1, PDL2, VISTA, BTNL2, B7-H3, B7-H4, CD48, HVEM, CD40L, TNFRSF25, GITR, 4-188, OX40, CD27, TMIGD2, ICOS, CD28, TCR, LAG3, CTLA4, PD1, CD244, TIM3, BTLA, CD160, and LIGHT, which are immune checkpoint proteins.

Examples of the pathway protein expressed in a cancer cell include: EGFR (HER1), HER2, HER3, HER4, IGFR, and HGFR, which are cancer cell growth factors or cancer cell growth factor receptors; VEGF-A, VEGF-B, VEGF-C, VEGF-D, VEGF-E, VEGF-R, PIGF-1, and PIGF-2, which are cell surface antigens, vascular growth factors, or vascular growth factor receptors; and interferon, interleukin, G-CSF, M-CSF, EPO, SCF, EGF, FGF, IGF, NGF, PDGF, and TGF, which are cytokines or cytokine receptors.

Examples of the progression protein expressed in a cancer cell include ACTG2, ALDOA, APC, BRMS1, CADM1, CAMK2A, CAMK2B, CAMK2D, CCL5, CD82, CDKN1A, CDKN2A, CHD4, CNN1, CST7, CTSL, CXCR2, YBB, DCC, DENR, DLC1, EGLN2, EGLN3, EIF4E2, EIF4EBP1, ENO1, ENO2, ENO3, ETV4, FGFR4, GSN, HK2, HK3, HKDC1, HLA-DPB1, HUNKIL11, KDM1A, KISS1, LDHA, LIFR, MED23, MET, MGAT5, MAP2K4, MT3, MTA1, MTBP, MTOR, MYCL, MYH11, NDRG1, NF2, NFKB1, NME1, NME4, NOS2, NR4A3, PDK1, PEBP4, PFKFB1, PFKFB4, PGK1, PLAUR, PTTG1 RB1, RORB, SET, SLC2A1, SNRPF, SSTR2, TCEB1, TCEB2, TCF20, TF, TLR4, TNFSF10, TP53, TSHR, MMP, MMP2, MMP10, and HIF1, which are cancer progression markers.

### (Biomarker B)

The biomarker B is a biological substance such as a protein or a nucleic acid expressed in a non-diseased cell that is adjacent to or close to a diseased cell in a diseased tissue in which the biomarker A is expressed, the diseased tissue being collected from a patient. In the present invention, the "non-diseased cell adjacent to a diseased cell" means a non-diseased cell existing in direct contact with a diseased cell in a diseased tissue. The "non-diseased cell close to a diseased cell" means that an intercellular substance such as a collagen fiber or a reticular fiber may be sandwiched between a non-diseased cell and a diseased cell in a pathogenic tissue, and for example, means a non-diseased cell existing within 100 µm from a diseased cell. Examples of the non-diseased cell include a fibroblast, a reticular cell, a histocyte, a plasma cell, an endothelial cell, a leukocyte (a lymphocyte, a monocyte, a neutrophil, an acidophil, or a basophil), an adipocyte, and a mast cell, which constitute stroma such as a connective tissue, a blood vessel, or a nerve. The biomarker B is not particularly limited as long as being derived from a non-diseased cell, and one type of biological substance may be selected to be used as the biomarker B, or two or more types of biological substances may be selected to be used as the biomarker B.

The biomarker B is one of those that exhibit an interaction with a non-diseased tissue existing around a diseased tissue, which means that surroundings of the diseased tissue are indirectly affected by a change of the diseased tissue. When the interaction is known, for example, a region in which the amount of the biomarker A is large or small in the diseased tissue is identified from a result of detection of the biomarker A with fluorescent nanoparticles, and the biomarker B can be extracted from the region.

When the biomarker B is involved in a signal transduction pathway, the biomarker B affects delivery of a drug targeting the biomarker A in a diseased tissue. Therefore, the biomarker B can be selected based on a difference in drug distribution in the diseased tissue. That is, a region in which the amount of a drug targeting the biomarker A is large or small is identified from a result of detection of the drug with fluorescent nanoparticles, and the biomarker B existing in the region can be extracted.

Such a method for extracting and identifying the biomarker B can be performed by a method suitable for a purpose. For example, by obtaining a tissue section from a diseased tissue by Cryotome Sectioning, isolating a plurality of cells, comprehensively quantifying the biomarkers A and B existing in each of the cells and an administered drug, and then reconstructing a quantification value of each of the cells on the tissue, distribution of the biomarkers A and B and the drug on the tissue can be obtained as an image.

Examples of a method for such a purpose include an analysis method called micro-pharmacokinetics (PK), which is a useful method that can provide detailed pharmacokinetics of a target substance that cannot be analyzed by macro-pharmacokinetics in a microenvironment in a tissue. (see Fig. 1).

The biomarker B can be identified, for example, by identification based on mutation information in gene analysis. That is, the biomarker B can be identified based on the mutation information in gene analysis using a sequencer or the like.

There are about 80 types of non-diseased cell-derived biomarkers B identified in this way. The biomarker B is preferably a biomarker involved in an interferon signaling pathway, and is more preferably BST2, OAS1, OAS2, OAS3, IFIT1, IFIT2, XAF1, clusterin, DCLK1, or MX1. Any of these can be selected and used for the purposes of the present invention.

Bone marrow stromal antigen 2 (BST2) is also referred to as CD317 (Tetherin), and is a lipid raft-related protein expressed in a bone marrow stromal cell. By detecting a virus or the like, a signal pathway is activated and expression is increased. Virally, BST2 is a human cell protein that inhibits viral infection because BST2 prevents spread of viral particles from an infected cell. BST2 is said to be involved in an intercellular interaction such as cell adhesion or cell migration. For this reason, BST2 also functions as a protein that stabilizes a lipid raft structure. OAS1, OAS2, and OAS3 are oligoadenylic acid synthetases and are induced by interferon. IFIT1 and IFIT2 are induced by interferon and are involved in transport of mature mRNA and the like. XAF1 is a protein that is bonded to an apoptosis inhibitor and suppresses an inhibitory effect. Clusterin is a protein involved in inhibition of apoptosis, lipid transport, hormone transport, and the like. DCLK1 is one of serine/threonine kinases and is considered to be involved in a calcium signal transduction pathway. MX1 is a protein induced by interferon during viral infection.

As a non-diseased cell included in a diseased tissue, a stromal cell is known. Examples of a protein included in the stromal cell include membrane proteins as illustrated below, which are stromal cell markers. Specific examples of the stromal markers and main distributions thereof are described below.
CD106 (VCAM-1 and INCAM-110) ... an activated vascular endothelial cell and a dendritic cell;
CD109 (Platelet activation factor, 8A3, and E123) ... an activated T cell, a platelet, a vascular endothelium, a megakaryocyte, and CD34 + a progenitor cell subset;
CD140a (PDGF-R and PDGFR2)...a fibroblast, a megakaryocyte, a monocyte, an erythrocyte, a myeloid progenitor cell, and an endothelial cell;
CD140b (PDGF-R and PDGFR1) ... an endothelial cell and a stromal cell;
CD141 (Thrombomodulin) ... a vascular endothelium, a myeloid cell, a platelet, and a smooth muscle;
CD142 (Tissue Factor (TF) and Thromboplastin) ... an epithelial cell, an activated monocyte, and an activated vascular endothelium;
CD143 (angiotensin converting enzyme (ACE)) ... a vascular endothelium, an epithelial cell, and an activated macrophage;
CD144 (VE-Cadherin and Cadherin-5) ... a vascular endothelium;
CD145 (7E9 and P7A5) ... an endothelial cell;
CD146 (MUC18, s-endo, and Mel-CAM) ... a vascular endothelium, an activated T cell, and melanoma;
CD147 (Basigin, M6, and EMMRRIN) ... a leukocyte, an erythrocyte, a vascular endothelium, and a platelet;
CD201 (Endothelial Protein C Receptor (EPCR)) ... a vascular endothelium;
CD202 (TIE2 and TEK) ... a vascular endothelium and a hematopoietic stem cell subset;
CD280 (Endo180, TEM22, and uPARAP (uPAR-associated protein)) ... a myeloid progenitor cell, a fibroblast, an endothelial cell subset, and a macrophage subset;
CD299 (DC-SIGN-related and Liver/Lympho node specific ICAM3-grabbing nonintegrin (L-SIGN)) ... an endothelial cell;
CD309 (Vascular endothelial growth factor receptor 2 (VEGFR2) and KDR) ... an endothelial cell, a megakaryocyte, a platelet, and a stem cell subset;
CD317 (BST2 and HM1.24) ... a lipid raft
CD322 (Junctional adhesion molecule 2 (JAM2)) ... an endothelial cell, a monocyte, a B cell, and a T cell subset;
CD331 (Fibroblast growth factor receptor 1 (FGFR1)) ... a fibroblasts and an epithelial cell;
CD332 (FGFR2 and Keratinocyte growth factor receptor) ... an epithelial cell;
CD333 (FGFR3 and JTK4) ... a fibroblasts and an epithelial cell;
CD334 (FGFR4, JTK2, and TKF) ... a fibroblasts and an epithelial cell; and
CD339 (Jagged-1 and JAG1) ... a stromal cell and an epithelial cell.

When the biomarker B is a nucleic acid, the biomarker B is preferably any one of various RNAs such as mRNA, tRNA, siRNA, and non-cording-RNA derived from a genome in stroma of a diseased tissue, and is preferably a miRNA such as miR21, miR34a, miR197, miR200, miR513, miR-133a, miR-143, exosomal micro-RNA(miR-181c, miR-27b), let-7a, miR-122, or iR4717.

### <Drug>

The drug is not particularly limited, but is preferably a drug having an antitumor effect, cytotoxicity, an anti-angiogenic effect, or an anti-inflammatory therapeutic activity, preferably a molecular-targeted drug, and particularly preferably a molecular-targeted drug for cancer. Above all, the drug is particularly preferably an antibody drug.

Examples of the molecular-targeted drug for cancer include: afatinib, erlotinib, gefitinib, cetuximaz, and vanitumumaz which are EGFR inhibitors; crizotinib which is an ALK inhibitor; labatinib which is an EGFR/HER2 inhibitor; trastuzumab, trastuzumab emtansine, and bebatzumab which are HER2 inhibitors; axitinib, snitinib, sorafenib, bazovanib, and legoraphenib which are angiogenesis inhibitors; eberolimus and temsirolimusm which are mTOR inhibitors; imatinib, dasatinib, and nilotinib which are BCR-ABL inhibitors; ibritumomab tiuxetan, ofatumumab, rituximab, brentuximab vedotin, gemtuzumab ozogamicin, and mogamulizumab which are membrane differentiation antigen-targeted drugs; and an antibody-drug complex such as trastuzumab emtansine (trade name; Kadocyla) in which a cytotoxic substance emtansine is bonded to a humanized HER2 antibody trastuzumab (herceptin), brentuximab vedotin (trade name; Adcetris) in which a microtube inhibitor monomethylauristatin E is bonded to an anti-CD30 monoclonal (mouse human chimeric) antibody, or gemtuzumab ozogamicin (trade name; Mylotarg). The drug bonded to an antibody is not limited to the above.

### <Step A: method for acquiring information on expression of biomarker A>

A method for acquiring information on expression of the biomarker A is not particularly limited, but for example, the information on expression of the biomarker A can be acquired by immunohistological analysis, gene analysis such as PCR, Northern blotting, or DNA microarray, or protein analysis such as ELISA, Western blotting, or LC/MS.

In the immunohistological analysis method, continuous sections are cut out from a frozen sample, a paraffin-embedded sample, or the like of a diseased tissue acquired from a sample. Some of the continuous sections are subjected to hematoxylin-eosin staining, Masson's trichrome staining, and the like to prepare specimen samples for morphological observation, and some of the continuous sections are subjected to immunostaining for the biomarker A to prepare specimen samples. By comparing microscopic observation between the two types of specimen samples, the intensity of expression of the biomarker A derived from a diseased cell in a diseased tissue can be acquired.

When immunostaining is performed, a diaminobenzidine (DAB) staining method using a reaction between peroxidase and DAB is particularly preferable because of an excellent staining property. When the DAB staining method is used, the biomarker A is labeled with an enzyme (peroxidase) and then caused to react with a substrate diaminobenzidine (DAB) to generate a dye. As a result, a region around the biomarker A is stained brown.

In a conventional immunohistological analysis methods, it is difficult to forecast arrival of a drug targeting the biomarker A inside a tissue because micro-level analysis is required. However, according to the method of the present invention, by precisely analyzing a drug distribution in a diseased tissue in which the biomarker A is expressed using a fluorescence imaging-drug analysis method, the biomarker B in a non-diseased tissue can be found from a difference thereof. That is, in the method of the present invention, by applying biological characteristics that are at a semi-quantitative level in conventional mutual analysis of a diseased tissue and a non-diseased tissue to observation of a drug distribution after drug administration, and further to a method for identifying diversity of a diseased tissue, the biomarker B can be found.

### <Step B: acquisition of information on expression of biomarker B>

(1) When a correlation between arrival of a drug targeting the biomarker A inside a tissue and expression of the biomarker B is known
   Information on expression of the biomarker B is acquired by a similar operation to the method for acquiring information on expression of the biomarker A.
(2) When a correlation between arrival of a drug targeting the biomarker A inside a tissue and expression of the biomarker B is not known

The biomarker B having a correlation with arrival of a drug targeting the biomarker A inside a tissue is screened as described later, and then expression information is acquired in a similar manner as to the above (1).

In screening the biomarker B, first, a diseased tissue is treated with a drug targeting the biomarker A, and a difference in distribution of the drug due to arrival thereof inside the tissue is evaluated in detail. For the evaluation, it is preferable to treat the specimen sample with a drug labeled with a fluorescent substance or the like because the distribution of the drug due to arrival thereof inside the tissue can be easily grasped by microscopic observation. Examples of the fluorescent substance include fluorescent dyes and fluorescent nanoparticles such as a rhodamine-based dye molecule, a squarylium-based dye molecule, a cyanine-based dye molecule, an aromatic ring-based dye molecule, an oxazine-based dye molecule, a carbopyronine-based dye molecule, a pyromesene-based dye molecule, an Alexa Fluor (registered trademark, manufactured by Invitrogen)-based dye molecule, a BODIPY (registered trademark, manufactured by Invitrogen)-based dye molecule, a Cy (registered trademark, manufactured by GE Healthcare)-based dye molecule, a DY-based dye molecule (registered trademark, manufactured by DYOMICS GmbH), a HiLyte (registered trademark, manufactured by AnaSpec)-based dye molecule, a DyLight (registered trademark, manufactured by Thermofisher Scientific)-based dye molecule, an ATTO (registered trademark, manufactured by ATTO-TEC GmbH)-based dye molecule, and an MFP (registered trademark, manufactured by Mobitec)-based dye molecule. Fluorescent nanoparticles are particularly preferable in terms of high brightness. As a method for labeling a drug with the fluorescent substance, a known method can be used. In order to identify a region in which a non-diseased cell adjacent to or close to a diseased tissue exists, the continuous sections used at the time of preparing the specimen samples of the above drug distribution are stained with hematoxylin-eosin, for example. In a region in which a distribution region of the drug labeled with the fluorescent substance and a region in which a non-diseased cell exists overlap each other, a region in which the drug arrival is relatively high is referred to as a drug high arrival region, and a region in which the drug arrival is relatively small is referred to as a drug low arrival region.

Subsequently, each of the regions is cut out from the specimen sample by a method such as microdissection, and gene information expressed in the region is acquired using, for example, DNA microarray or mRNA microarray. From the acquired gene information, for example, by GO analysis, a function of an expressed gene is roughly estimated by GO Term. Among these GO Terms, top two or three GO Terms that have been upregulated (hereinafter referred to as "high GO Term") are selected, and genes with high expression ratios are examined in each of the GO Terms. A gene highly expressed commonly in the GO Terms is referred to as a candidate biomarker B.

Subsequently, in order to examine a relationship between the candidate biomarker B and the drug targeting the biomarker A, an effective amount of the drug is administered to a model animal or the like forming the diseased tissue, and the diseased tissue is collected after a predetermined period of time. A continuous section cut out from a frozen sample of the collected diseased tissue is prepared, and a gene expressed in a drug high arrival region is analyzed in a similar manner to the above. As a result, when a p-value increases or decreases in the previous high GO Term and this high GO Term, it is estimated that the expression level of the candidate biomarker B has been affected by administration of the drug targeting the biomarker A.

In order to examine a relationship between the biomarker A and the candidate biomarker B, for example, the specimen sample prepared from the continuous section prepared by administering the drug to the above model animal is subjected to immunostaining for the biomarker A and the candidate biomarker B. Thereafter, when the same region is observed with a microscope and a positive or negative correlation can be confirmed between expression of the biomarker A and expression of the candidate biomarker B, the candidate biomarker B can be used as the target biomarker B. Presence or absence of a relationship between the biomarker A and the candidate biomarker B can be examined by quantitatively measuring the expression levels of genes of both the biomarkers, for example, the expression levels of mRNA.

### <Acquisition of information forecasting drug arrival>

When a correlation between arrival of a drug targeting the biomarker A inside a tissue and expression of the biomarker B is known, arrival of the drug targeting the biomarker A inside the tissue can be forecasted in advance from the information on expression of the biomarker B obtained by the method of step B depending on whether the biomarker B is strongly expressed or weakly expressed. Therefore, for example, only by gene polymorphism analysis, immunohistochemical observation, or acquisition of the expression level of mRNA, information forecasting arrival of the drug targeting the biomarker A inside the tissue can be acquired.

When a correlation between arrival of a drug targeting the biomarker A inside a tissue and expression of the biomarker B is not known, first, information on expression of the biomarker A by step A is acquired, and the drug targeting the biomarker A is identified. Next, the information on expression of the biomarker B by step B is acquired, and the correlation between arrival of the drug inside the tissue and expression of the biomarker B is examined. Thereafter, according to the correlation, information forecasting arrival of the drug targeting the biomarker A inside the tissue can be acquired from the intensity of expression of the biomarker B. Note that even for the drug targeting the biomarker A, the intensity level of the correlation of the level of arrival of the drug inside the tissue in involvement of biomarker B is not particularly limited.

### <Drug screening>

As described above, the biomarker B involved in a signal transduction pathway affects delivery of the drug targeting the biomarker A in a diseased tissue. Therefore, in a diseased tissue in which the biomarker A derived from a diseased cell is expressed, the information on expression of the biomarker B derived from a non-diseased cell adjacent to or close to the diseased cell described above is acquired, and the drug targeting the biomarker A can be screened based on the information forecasting arrival of the drug targeting the biomarker A inside the diseased tissue.

### <Activity regulator and pharmaceutical composition>

Another aspect of the present invention is an activity regulator that suppresses or promotes activity of the biomarker B involved in drug delivery in a pharmaceutical composition targeting the biomarker A. Another aspect of the present invention is a pharmaceutical composition containing the activity regulator. The pharmaceutical composition can be in forms of various processed preparations. Examples thereof include a parenteral preparation such as an infusion, a nasal drop, or an injection. The pharmaceutical composition may further contain an additive commonly used in the pharmaceutical field. Examples of such an additive include an antioxidant, a colorant, and a suspending agent, which can be blended as long as the effects of the present invention are not impaired.

### Examples

The present invention will be described more specifically based on Examples, but is not limited to these Examples.

### [Preparation Example 1]

### <Preparation of diseased tissue model sample>

### (1) Preparation of CDX sample

Human breast cancer cell lines BT-474 and HCC1954 were purchased from the American Type Culture Collection (ATCC). BT474 and HCC1954 were cultured in an RPMI-1640 medium containing 10% Fetal Bovine Serum (FBS) and 1% penicillin and streptomycin.

Each of the cultured cancer cell lines was subjected to trypsin treatment and then dispersed in a PBS buffer. An equal amount of Matrigel (registered trademark) (product number: 356231, manufactured by Corning Inc.) was added thereto and stirred to obtain a cell suspension. A part of the cell suspension was set aside for preparation such that the concentration of the cell suspension was 1.0 × 10⁷ cells/100 µL to be used as a transplantation suspension. The transplantation suspension was inoculated into the left flank of a 4-week-old female immunodeficient mouse (SCID-Beige: CB17. Cg-PrkdcscidLystbg-J/CrlCrlj, purchased from Charles River Laboratories Japan, Inc.). When the inoculated cancer cell reached a size of 150 to 200 mm³, trastuzumab (1 mg/kg or 10 mg/kg) or trastuzumab emtansine (10.2 mg/kg) was intraperitoneally administered. After a lapse of a predetermined time after the administration, the mouse was euthanized, and the cancer cell was collected to be used as a CDX sample of BT474 or a CDX sample of HCC1954.

### (2) Preparation of animal model sample

A cancer tissue (PDX) derived from a breast cancer patient was cut into fragments of 1 mm³ or less and transplanted into a breast fat pad of a 4-week-old female immunodeficient mouse (SCID-Beige). The mouse was used as a first-generation transplanted mouse. After the cancer tissue transplanted into the first-generation transplanted mouse grew to a predetermined size, the grown cancer tissue was transplanted into another immunodeficient mouse. The mouse was used as a second-generation transplanted mouse. This transplantation operation was repeated to prepare a fourth-generation transplanted mouse. When the cancer cell inoculated into the fourth-generation transplanted mouse reached a size of 150 to 200 mm³, trastuzumab (1 mg/kg or 10 mg/kg) was intraperitoneally administered. After a lapse of a predetermined time after the administration, the mouse was euthanized, and the cancer cell was collected to be used as an animal model sample of No. 1 or No. 2.

### (3) Preparation of specimen slide

The two types of CDX samples (BT474 and HCC1954) and the two types of PDX samples (No. 1 and No. 2) prepared above were each embedded in a frozen tissue embedding agent and frozen with liquid nitrogen/n-hexane to prepare frozen blocks. Each of the frozen blocks was sliced into a thickness of 8 µm using a cryomicrotome (product code: CM1950, manufactured by Leica Biosystems) to prepare a frozen continuous section, and the frozen continuous section was attached to a slide glass. The section was immersed in a 4% paraformaldehyde fixative for three minutes to be fixed, and then immersed in RNase-free ice-cold water for one minute to remove an OCT compound. Thereafter, endogenous peroxidase was blocked with 0.3% hydrogen peroxide dissolved in TBS to prepare each specimen slide.

### [Example 1]

### <Acquisition of trastuzumab arrival information inside tissue>

### (1) Fluorescent staining treatment with high-brightness fluorescent nanoparticles

The two types of PDX (No. 1 and No. 2) specimen slides prepared in Preparation Example 1 were each washed with a PBS buffer. A solution obtained by diluting high-brightness fluorescent nanoparticles to which an anti-trastuzumab antibody was bonded (manufactured by Konica Minolta Co., Ltd., hereinafter referred to as "trastuzumab fluorescent nanoparticles") to 0.02 nM with a diluent (casein: BSA = 5%) was added dropwise thereto. The resulting mixture was caused to react at room temperature for three hours in a neutral pH environment (pH 6.9 to 7.4) to be subjected to trastuzumab fluorescent nanoparticle treatment.

### (2) Staining for morphological observation

The specimen slide that had been subjected to fluorescent staining treatment was stained with a hematoxylin liquid (manufactured by Fuji Film Wako Pure Chemical Industries, Ltd.) and an eosin liquid (manufactured by Fuji Film Wako Pure Chemical Industries, Ltd.) for five minutes according to a conventional method to be subjected to hematoxylin-eosin staining. Thereafter, the resulting product was washed with running water at 45°C for three minutes.

### (3) Encapsulation treatment

An operation of immersing the stained specimen slide in pure ethanol for five minutes was performed four times to perform fixation and dehydration treatment. Subsequently, an operation of immersing the specimen slide in xylene for five minutes was performed four times to perform permeation treatment. Thereafter, an encapsulant "Entellan New" (manufactured by Merck & Co.) was placed on the specimen. The specimen was covered with a cover glass to be subjected to encapsulation treatment, and used as an observation specimen.

### (4) Observation and imaging

A BX63 fluorescence microscope (manufactured by Olympus Corporation) was used for fluorescence observation and imaging. First, the observation specimen was irradiated with excitation light corresponding to Texas red contained in trastuzumab fluorescent nanoparticles to emit fluorescence, and an immunostaining image was taken in this state. At this time, the excitation light was set to 575 to 600 nm using an optical filter for excitation light, and a wavelength to be observed was set to 612 to 692 nm using an optical filter for fluorescence. Results thereof are illustrated in Fig. 2. Fig. 2 indicates that in either specimen, more bright spots of trastuzumab fluorescent nanoparticles are observed inside a connective tissue region, that is, inside a non-diseased tissue adjacent to a cancer cell region (connective tissue region in Fig. 2A) than inside the cancer cell region, that is, inside a diseased tissue ("cancer cell region" in Fig. 2A) (Fig. 2B). A region with fewer trastuzumab fluorescent nanoparticles in a specimen is referred to as "trastuzumab low arrival region", and a region with more trastuzumab fluorescent nanoparticles is referred to as "trastuzumab high arrival region".

### [Example 2]

### <Acquisition of information on expression of biomarker B>

### (1) Laser microdissection

In the PDX (NO. 2) specimen prepared in Example 1, from the trastuzumab high arrival region and the trastuzumab low arrival region, a trastuzumab high arrival region section and a trastuzumab low arrival region section were cut out, respectively using a laser microdissection device (trade name: LMD6500, manufactured by Leica Microsystems), and put in a collection microtube containing an RLT buffer attached to an RNA extraction kit described later to be collected.

### (2) Extraction of mRNA

mRNA of each section that had been cut out was performed using an RNA extraction kit "RNeasy (registered trademark) Micro kit" (manufactured by QIAGEN) according to the instructions attached to the kit.

### (3) Microarray and GO analysis

The Chemicals Evaluation and Research Institute was commissioned to perform microarray analysis on the extracted mRNA using a human gene expression level microarray chip "SurePrint G3 Human GE Microarray 8 x 60 K" (manufactured by Agilent Technologies). Data analysis was performed using Genespring softwere (manufactured by Agilent Technologies), and data mining (annotation) was performed using Database for Annotation, Visualization and Integrated Discovery (DAVID) v 6.8. Thereafter, gene ontology analysis (GO analysis) was performed on 442 genes expressed in the trastuzumab high arrival region 1.5 times more or 0.67 times less than in the trastuzumab low arrival region. Results thereof are illustrated in Fig. 3. Fig. 4 illustrates results of examining the mRNA expression levels in Type I interferon signaling and Response to virus, which were the highest in the GO Terms in Fig. 3. mRNA expression of BST2 was the highest in either GO Term.

### [Example 3]

In order to examine a relationship between expressions of HER2 and BST2 and trastuzumab arrival information inside a tissue, each piece of information was acquired as follows.

### <Acquisition of HER2 expression information>

### (1) Primary antibody treatment

Each of the tissue sections of the specimen slides of the two types of CDX samples (BT474 and HCC1954) and the two types of PDX samples (No. 1 and No. 2) prepared in Preparation Example 1 above was immunostained as follows.

Each of the tissue sections was incubated with a permeation treatment liquid (0.1% Triton (registered trademark) X and 5% goat serum in a Tris-Buffer Saline (TBS) buffer) at room temperature for one hour to be subjected to permeation treatment. The tissue section was caused to react with an anti-HER2 rabbit monoclonal antibody (product number: 4290, manufactured by Cell Signaling Technology), which is a primary antibody diluted 100 times with a diluent attached to the product, overnight at 4°C.

### (2) Secondary antibody treatment

The tissue section that had reacted was washed with a TBS buffer. Thereafter, the tissue section was caused to react with a secondary antibody (product number: 8114, manufactured by Cell Signaling Technology) at room temperature. The tissue section was washed with a TBS buffer. Thereafter, a DAB substrate (product number: 8059, manufactured by Cell Signaling Technology) was added to the tissue section to cause the tissue section to develop color.

### (3) Encapsulation treatment

The stained specimen slide was subjected to dehydration treatment with ethanol according to a conventional method. Thereafter, the specimen slide was immersed in xylene to be subjected to permeation treatment. Finally, an encapsulant "Entellan New" was placed on the specimen, covered with a cover glass to be subjected to encapsulation treatment, and used as an observation specimen sample.

### <Acquisition of BST2 expression information>

The tissue section of each of the observation specimen samples was subjected to primary staining with an anti-BST2 mouse monoclonal antibody (Product No.: 557355, BD Pharmingen) in a similar manner to the above immunostaining of HER2. Thereafter, secondary antibody treatment and encapsulation treatment were performed in a similar manner to the above to prepare an observation specimen sample.

### <Acquisition of trastuzumab arrival information inside tissue>

In a similar manner to Example 1, the tissue section of each of the observation specimen samples was subjected to trastuzumab fluorescent nanoparticle staining and encapsulation treatment.

### <Observation and imaging>

BZ-X710 (manufactured by Keyence Co., Ltd.) was used for observing the above observation specimen samples for HER2 and BST2, and Nano Zoomer S60 (manufactured by Hamamatsu Photonics Co., Ltd.) was used for taking a fluorescent immunostaining image and a morphological observation staining image. The observation specimen sample for trastuzumab was observed in a similar manner to Example 1. Results thereof are illustrated in Fig. 5. In Fig. 5, in the specimen sample for BT474, BST2 expression is very low, but HER2 expression is strong, while in the PDX (NO. 2) specimen sample, BST2 expression is very strong, but HER2 expression is weak. From this result, it can be considered that the biomarker B such as BST2 expressed in a non-diseased tissue existed around a tumor tissue and was affected by the tumor tissue to exhibit a negative correlation with expression of the biomarker A such as HER2. In addition, it was also observed that a fluctuation in the number of trastuzumab bright spots in a tissue exhibited a negative correlation with BST2 expression.

Furthermore, micro imaging was performed with the concept illustrated in Fig. 1. As a result, from the PDX (NO. 1) specimen sample, it has been confirmed that the arrival level of trastuzumab inside a tissue is low (the number of bright spots is small) in any region where BST2 expression is relatively strong (Fig. 6A), and that the arrival level of trastuzumab inside a tissue is high (the number of bright spots is large) in any region where BST expression is relatively low (Fig. 6B). By the way, in micro imaging, when HER2 expression was observed in several regions in the PDX (NO. 1) specimen sample and the PDX (NO. 2) specimen sample, it was confirmed that no clear correlation between HER2 expression and the arrival level of trastuzumab inside a tissue was observed. This suggests that micro imaging may be essential in a process of achieving the present invention.

### [Example 4] <Trends of HER2 expression in BST2 knockout tumor cell transplanted mouse>

100 nM OnTarget (registered trademark) siRNA designed to target BST2 or siRNA (manufactured by Horizon Discovery) as a control was introduced into HCC-1954-luciferase cells and cultured in an RPMI-1640 medium. After culturing, the cells were subjected to trypsin treatment according to a conventional method and suspended in a PBS buffer. Thereafter, 1/10 amounts of Matrigel (registered trademark) and 100 nM Accell (registered trademark) siRNA designed to target BST2 or siRNA (manufactured by Horizon Discovery) as a control were added thereto to prepare a cell suspension. A 1.7 × 10⁷ cells/100 µL cell suspension was inoculated subcutaneously into the left flank of a 4-week-old female immunodeficient mouse (SCID-Beige).

About one week after the inoculation, when a cancer cell reached a size of 150 to 200 mm³, trastuzumab (1 mg/kg or 10 mg/kg) was intraperitoneally administered. After a lapse of a predetermined time after the administration, the mouse was euthanized, and the cancer cell was collected to prepare a BST2 knockout tumor transplantation model.

Using the PDX, an observation specimen sample for HER2 expression was prepared in a similar manner to Example 3, and morphologically observed. Results thereof are illustrated in Fig. 7. In the BST2 knockout tumor transplantation model ("BST2KD" in Fig. 7), it was observed that BST2 expression was suppressed and strong HER2 expression did not change, while in the control PDX ("HCC1954 wild" in Fig. 7A"), it was observed that BST2 expression was reduced and delivery of an anti-HER2 drug trastuzumab inside a tissue was suppressed. In addition, in the BST2 knockout tumor transplantation model, the number of bright spots indicating trastuzumab per cell was significantly larger than that of the control PDX ("Wild" in Fig. 7B). That is, it has been indicated that BST2 is involved in arrival of trastuzumab inside a cancer tissue.

From the results of Examples 1 to 4 above, it has been indicated that when BST2 expression is strong, arrival of trastuzumab inside a cancer tissue is difficult regardless of HER2 expression, which is one of the biomarkers A. Conversely, it has been indicated that when BST2 expression is weak, arrival of trastuzumab inside a cancer tissue is easy. Therefore, by examining BST2 expression information, it is possible to forecast arrival of trastuzumab targeting HER2 inside a cancer tissue.

### [Reference Example]

From genome-wide microarray and survivor information of 1809 breast cancer patients obtained from Gene Expression Omnibus, a progression-free survival ratio due to a difference in BST2 expression was determined by a Kaplan-Meier method, which is illustrated in Fig. 8. As a result, it has been found that the stronger the BST2 expression (lower graph of Fig. 8) is, the lower the survival ratio is as compared with a case where the BST2 expression is weak (upper graph of Fig. 8). From this fact and the results of Examples 1 to 4, BST2 can be used as a biomarker for estimating therapeutic efficiency using trastuzumab, and a relationship between a tumor resistance mechanism and drug delivery can be clarified, which can be used for developing a novel drug.

In addition, the present invention has been completed, for example, from a micro-PK analysis method for quantifying the biomarkers A and B existing in a diseased tissue and a molecular-targeted drug targeting the biomarker A and elucidating distributions thereof. When an example of the analysis result described herein is further considered, the results of quantification and distribution analysis of the biomarker B suggest that the state of a disease is closely related to the biomarker B, and a drug that acts to enhance or limit the function of the biomarker B may be a drug with effective efficacy.

## Claims

1. A method for forecasting,
in a diseased tissue in which a biomarker A derived from a diseased cell is expressed,
arrival of a drug targeting the biomarker A inside the diseased tissue, the method comprising
a step B for acquiring information on expression of a biomarker B derived from a non-diseased cell adjacent to or close to the diseased cell.

2. A method for forecasting arrival of a drug targeting a biomarker A derived from a diseased cell in a diseased tissue inside the diseased tissue from both of information on expression of the biomarker A and information on expression of a biomarker B derived from a non-diseased cell adjacent to or close to the diseased cell, the method comprising: a step A for acquiring the information on expression of the biomarker A; and a step B for acquiring the information on expression of the biomarker B.

3. The method according to claim 1 or 2, wherein the diseased tissue is a cancer tissue, and the diseased cell is a cancer cell.

4. The method according to any one of claims 1 to 3, wherein the biomarker A is at least one selected from the group consisting of an immune protein expressed in a cancer cell, a pathway protein expressed in a cancer cell, and a progression protein expressed in a cancer cell.

5. The method according to any one of claims 1 to 4, wherein the biomarker B is a protein involved in an interferon signaling pathway and is at least one selected from the group consisting of BST2, OAS1, OAS2, OAS3, IFIT1, IFIT2, XAF1, clusterin, DCLK1, and MX1.

6. The method according to any one of claims 1 to 5, wherein the drug is a molecular-targeted drug.

7. A method for screening a drug targeting a biomarker A derived from a diseased cell in a diseased tissue based on the method for forecasting arrival of the drug targeting the biomarker A inside the diseased tissue according to any one of claims 1 to 6.

8. An activity regulator of a biomarker B derived from a non-diseased cell adjacent to or close to a diseased cell.

9. A pharmaceutical composition comprising a drug targeting a biomarker A derived from a diseased cell in a diseased tissue and the activity regulator according to claim 8.
